# EUROPEAN PATENT APPLICATION

(11) **EP 4 817 644 A1**
(43) Date of publication of application: **07.10.2026**
(21) Application number: 24904426.4
(22) Date of filing: 13.12.2024
(51) Int. Cl.: A61L 27/52, A61L 27/18

(54) **HYDROGEL COMPOSITION FOR HUMAN BODY ULTRASONOGRAPHY SIMULATION, PREPARATION METHOD THEREFOR, AND ARTIFICIAL ORGAN COMPRISING TISSUE FORMED THEREFROM**

(30) Priority: 14.12.2023 KR 20230181430
(71) Applicant: Aldaver Inc., Daejeon 34051 (KR)
(72) Inventor: LEE, Ye Rim, Daejeon 35202 (KR); KIM, Jin-Oh, Daejeon 34040 (KR); SON, Ryeo Jin, Daejeon 34052 (KR)
(74) Representative: dompatent
(86) International application number: PCT/KR2024/096920
(87) International publication number: WO 2025/127861

(57) **Abstract**

The present invention relates to a hydrogel composition for an ultrasonography simulation, a preparation method therefor, and an artificial organ comprising tissue formed therefrom, and more specifically to a hydrogel composition for human body ultrasonography simulation, a preparation method therefor, and an artificial organ comprising tissue formed therefrom.

## Description

### [Technical Field]

This application claims the benefit of priority based on Korean Patent Application No. 10-2023-0181430 filed on December 14, 2023, all contents of which are incorporated as part of this specification.

The present invention relates to a hydrogel composition for an ultrasonography simulation, a preparation method therefor, and an artificial organ comprising a tissue formed from the hydrogel composition, and more specifically, to a hydrogel composition for a human body ultrasonography simulation, a preparation method therefor, and an artificial organ comprising a tissue formed from the hydrogel composition.

### [Background Art]

Compared to the steadily increasing number of patients requiring organ transplants, the supply of available organs remains limited. To address this, there is a growing need for the development of artificial organs or artificial tissues, and active research is underway regarding the manufacture of hydrogel-based artificial organs or tissues that minimize immune rejection and foreign body sensation when implanted in the body.

Furthermore, ultrasonography is a method of examining abnormalities in body tissues using ultrasound waves, and more specifically, it is a diagnostic method that transmits ultrasound waves into the body part to be measured and then acquires the reflected images as an image to determine the presence of abnormal tissue. Currently, ultrasonography is widely used in most medical examinations, not only for the diagnosis of lesions such as tumors or fetuses but also for general medical testing.

However, conventional hydrogel artificial organs or tissues appear black without color distinction during ultrasound measurements, which reduces the usability of the artificial organs or tissues. Therefore, there is a need to develop hydrogel materials for human body ultrasonography simulation that can overcome this problem.

### [Prior Art Document]

### [Patent Document]

(Patent Document 1) Korean Patent Publication No. 10-1725311(2017.04.04.)

### [Disclosure]

### [Technical Problem]

To resolve the above-mentioned problems of the prior art,
it is an object of the present invention to provide a hydrogel composition for an ultrasonography simulation that is useful for manufacturing an artificial organ or artificial tissue for implantation in the body.

It is an another object of the present invention to provide a hydrogel composition for an ultrasonography simulation in which each tissue is distinctly rendered during ultrasonography observation, allowing it to be observed like an actual human organ or tissue.

It is an another object of the present invention to provide an artificial organ comprising a tissue formed from the hydrogel composition for an ultrasonography simulation.

It is an another object of the present invention to provide a method for efficiently preparing the hydrogel composition for an ultrasonography simulation.

### [Technical Solution]

To achieve the above objects, the present invention
provides a hydrogel composition for an ultrasonography simulation comprising a hydrogel monomer and a water-dispersible fibrous material, wherein the content of the fibrous material is 0.001 wt% to 5.0 wt% based on the total weight of the composition.

In addition, the present invention provides a hydrogel composition for an ultrasonography simulation in which the hydrogel monomer is at least one selected from acrylamide, agarose, methacrylamide, bis-acrylamide, N-N-dimethylacrylamide, N,N-methylenebisacrylamide, polyaniline, acrylic acid, methacrylic acid, vinylsulfonic acid, styrenesulfonic acid, polyacrylic acid, allylamine, methyl methacrylate, poly(ethylene glycol) methacrylate, poly(ethylene glycol) diacrylate, sodium polyacrylate, N-vinylpyrrolidone, imide, amide, agarose, gelatin, gelatin methacrylate, collagen, fibrinogen, fibrin, methyl cellulose, chitosan, chitin, polyethylene glycol, polyvinyl alcohol, carrageenan, 2-acrylamido-2-methylpropane-sulfonic acid, alginate, 2-hydroxyethyl methacrylate, and mixtures thereof.

In addition, the present invention provides a hydrogel composition for an ultrasonography simulation in which the fibrous material is at least one selected from cellulose, cellulose nanofibers, glass fibers, and silicon oxide (SiO₂).

In addition, the present invention provides a hydrogel composition for an ultrasonography simulation comprising 10 to 25 wt% of acrylamide, 1 to 4 wt% of agarose, 0.001 to 5.0 wt% of cellulose, 0.005 to 0.03 wt% of N,N-methylenebisacrylamide (MBA), 0.05 to 0.10 wt% of ammonium persulfate (APS), and 70 to 90 wt% of water, based on the total weight of the composition.

In addition, the present invention provides an artificial organ comprising a tissue formed from the hydrogel composition for an ultrasonography simulation.

The artificial organ may be an artificial kidney, artificial liver, artificial small intestine, artificial large intestine, artificial stomach, artificial gallbladder, artificial blood vessel, artificial bladder, artificial heart, artificial lung, artificial uterus, artificial skin, or lymph node.

In addition, the present invention provides a preparation method for a hydrogel composition for an ultrasonography simulation comprising the steps of: (a) dissolving 10 to 25 wt% of acrylamide and 1 to 4 wt% of agarose in 70 to 90 wt% of water, based on the total weight of the composition; (b) adding 0.001 to 5.0 wt% of cellulose to the dissolved solution of the step (a) and dispersing it; and (c) adding 0.005 to 0.03 wt% of N,N-methylenebisacrylamide (MBA) and 0.05 to 0.10 wt% of ammonium persulfate (APS) to the dispersed solution of the step (b) and dissolving them.

### [Advantageous Effects]

The hydrogel composition for an ultrasonography simulation of the present invention can be usefully employed in the manufacture of artificial tissues or artificial organs for implantation in the body.

In addition, the artificial tissue formed from the hydrogel composition for an ultrasonography simulation of the present invention is realized such that each tissue is distinguishable upon ultrasonography observation, allowing it to be observed like actual human tissue or organ.

Furthermore, the present invention provides a preparation method for a hydrogel composition for an ultrasonography simulation that is observed like actual human tissue or organ upon ultrasonography observation.

### [Description of Drawings]

FIG. 1 is a diagram showing an ultrasonic image of actual human skin according to the prior art.
FIG. 2 is a diagram showing an ultrasonic image of a hydrogel according to one embodiment of the present invention.
FIG. 3 is a diagram showing an ultrasonic image of a hydrogel according to one embodiment of the present invention.

### [Best Mode]

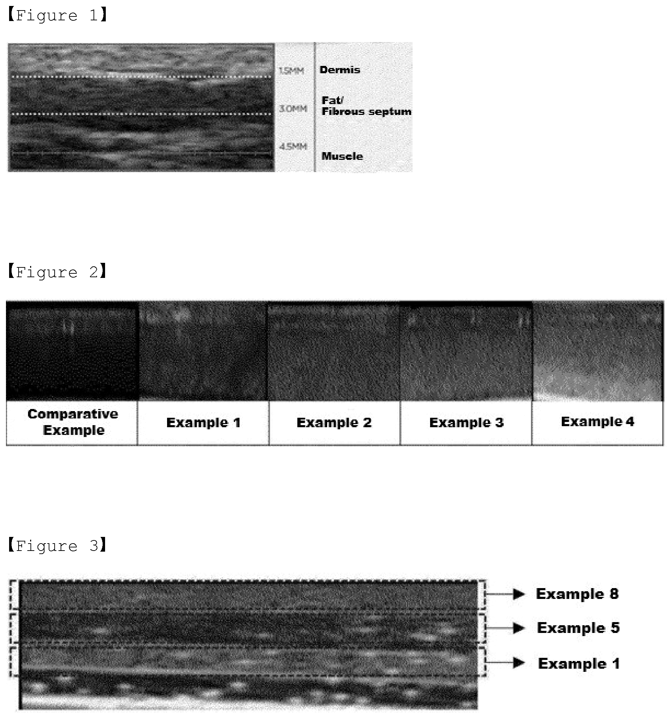

FIG. 1 is a diagram showing an ultrasonic image of actual human skin according to the prior art. As shown in the image of FIG. 1, actual human skin has different amounts of reflection for each tissue, so it can be seen that the degree to which it appears white to the naked eye varies as a result of ultrasound imaging.

However, conventional artificial tissues or artificial organs made of hydrogel material were observed as black without color distinction during ultrasound measurement, which had the problem of reducing the usability of the artificial tissues or artificial organs.

Accordingly, the inventors of the present invention have developed a hydrogel for an ultrasonography simulation that distinguishes each tissue when observed by ultrasound, making it appear like actual human tissue or organ.

In particular, the present invention aims to provide a hydrogel that allows each tissue to be distinguished when observing all artificial organs and artificial tissues, such as lymph nodes, lesions, artificial kidneys, and artificial skin, by ultrasound.

Hereinafter, the present invention will be described in detail.

According to one embodiment of the present invention, a hydrogel composition for an ultrasonography simulation comprises a hydrogel monomer and a water-dispersible fibrous material, and the content of the fibrous material may be 0.001 wt% to 5.0 wt% based on the total weight of the composition.

The hydrogel monomer is one of the components constituting a hydrogel for ultrasonography simulation, and may be at least one selected from acrylamide, agarose, methacrylamide, bis-acrylamide, N-N-dimethylacrylamide, N,N-methylenebisacrylamide, polyaniline, acrylic acid, methacrylic acid, vinylsulfonic acid, styrenesulfonic acid, polyacrylic acid, allylamine, methyl methacrylate, poly(ethylene glycol) methacrylate, poly(ethylene glycol) diacrylate, sodium polyacrylate, N-vinylpyrrolidone, imide, amide, agarose, gelatin, gelatin methacrylate, collagen, fibrinogen, fibrin, methyl cellulose, chitosan, chitin, polyethylene glycol, polyvinyl alcohol, carrageenan, 2-acrylamido-2-methylpropane-sulfonic acid, alginate, 2-hydroxyethyl methacrylate and mixtures thereof.

The fibrous material is a water-dispersible material and may be at least one selected from cellulose, cellulose nanofibers, glass fibers, and silicon oxide (SiO₂).

The content of the fibrous material may be 0.001 wt% to 5.0 wt% based on the total weight of the composition. When the fibrous material is included in an amount of less than 0.001 wt% based on the total weight of the composition, the prepared hydrogel appears black without color distinction during ultrasonic observation, which reduces its usability as an artificial organ or tissue. Additionally, when the fibrous material is included in an amount exceeding 5.0 wt% based on the total weight of the composition, the prepared hydrogel may appear white like actual tissue during ultrasonic observation, but it is difficult to distinguish the color, which reduces its usability as an artificial organ or tissue.

According to one embodiment of the present invention, a hydrogel composition for an ultrasonography simulation may comprise, based on the total weight of the composition, 10 to 25 wt% of acrylamide, 1 to 4 wt% of agarose, 0.001 to 5.0 wt% of cellulose, 0.005 to 0.03 wt% of N,N-methylenebisacrylamide (MBA), 0.05 to 0.10 wt% of ammonium persulfate (APS), and 70 to 90 wt% of water.

When the acrylamide is included in an amount of less than 10 wt%, there is a problem that the hydrogel does not cure sufficiently. In addition, when the acrylamide is included in an amount exceeding 25 wt%, the modulus becomes too large, resulting in physical properties different from those of actual organs, which makes it difficult to use as an artificial organ or artificial tissue.

Accordingly, it is preferable that the acrylamide be included in an amount of 10 to 25 wt% based on the total weight of the composition, and more specifically, based on the total weight of the composition, it may be 10 wt% or more, 11 wt% or more, 12 wt% or more, 13 wt% or more, 14 wt% or more, 15 wt% or more, 16 wt% or more, 17 wt% or more, or 25 wt% or less, 24 wt% or less, 23 wt% or less, 22 wt% or less, 21 wt% or less, or 20 wt% or less.

When the agarose is included in an amount of less than 1 wt%, the mechanical properties of the hydrogel become too weak, causing it to tear and break easily. In addition, when the agarose is included in an amount exceeding 4 wt%, the agarose does not dissolve well in water, making it difficult to prepare the hydrogel.

Accordingly, it is preferable that the agarose be included in an amount of 1 to 4 wt% based on the total weight of the composition, and more specifically, in an amount of 1 wt% or more, 1.2 wt% or more, 1.4 wt% or more, 1.6 wt% or more, or 4 wt% or less, 3.8 wt% or less, 3.6 wt% or less, 3.4 wt% or less, or 3.2 wt% or less, based on the total weight of the composition.

When the cellulose is included in an amount of less than 0.001 wt%, the reflection and scattering effects of particle ultrasound are negligible when the prepared hydrogel is observed ultrasonically, so it appears black without color distinction, which reduces its usability as an artificial organ or tissue. In addition, when the cellulose is included in an amount exceeding 5.0 wt%, although the prepared hydrogel may appear white like actual tissue when observed ultrasonically, the signal becomes irregular due to excessive reflection and scattering effects of ultrasound caused by the high content in the overall composition, and it is difficult to distinguish colors, which reduces its usability as an artificial organ or tissue.

Accordingly, it is preferable that the cellulose be included in an amount of 0.001 to 5.0 wt% based on the total weight of the composition, and more specifically, in an amount of 0.001 wt% or more, 0.003 wt% or more, 0.005 wt% or more, 0.007 wt% or more, 0.009 wt% or more, 0.01 wt% or more, 0.02 wt% or more, 0.03 wt% or more, 0.04 wt% or more, 0.05 wt% or more, 0.06 wt% or more, 0.07 wt% or more, 0.08 wt% or more, 0.09 wt% or more, 0.10 wt% or more, 0.12 wt% or more, 0.14 wt% or more, 0.16 wt% or more, 0.18 wt% or more, 0.20 wt% or more, 0.22 wt% or more, 0.24 wt% or more, 0.26 wt% or more, 0.28 wt% or more, 0.30 wt% or more, or 5.0 wt% or less, 4.8 wt% or less, 4.6 wt% or less, 4.4 wt% or less, 4.2 wt% or less, 4.0 wt% or less, 3.8 wt% or less, 3.6 wt% or less, 3.4 wt% or less, 3.2 wt% or less, or 3.0 wt% or less, based on the total weight of the composition.

When the N,N-methylenebisacrylamide (MBA) is included in an amount of less than 0.005 wt%, crosslinking with acrylamide is not sufficiently achieved, making it difficult to prepare the hydrogel. In addition, when the N,N-methylenebisacrylamide (MBA) is included in an amount exceeding 0.03 wt%, the degree of chemical crosslinking increases, which reduces the toughness of the hydrogel, resulting in physical properties that differ from those of actual organs, making it difficult to use as an artificial tissue.

Accordingly, it is preferable that the N,N-methylenebisacrylamide (MBA) be included in an amount of 0.005 to 0.03 wt% based on the total weight of the composition, and more specifically, based on the total weight of the composition, it may be 0.005 wt% or more, 0.007 wt% or more, 0.009 wt% or more, 0.01 wt% or more, or 0.03 wt% or less, 0.028 wt% or less, 0.026 wt% or less, 0.024 wt% or less, 0.022 wt% or less, or 0.02 wt% or less.

When the ammonium persulfate (APS) is included in an amount of less than 0.05 wt%, there is a problem that the hydrogel curing time becomes prolonged. In addition, when the ammonium persulfate is included in an amount exceeding 0.10 wt%, it is undesirable because residual components not used for curing may remain, causing surface stickiness.

Therefore, it is preferable that the ammonium persulfate (APS) be included in an amount of 0.05 to 0.10 wt% based on the total weight of the composition, and more specifically, it may be 0.05 wt% or more, 0.06 wt% or more, 0.07 wt% or more, or 0.1 wt% or less, 0.09 wt% or less, or 0.08 wt% or less, based on the total weight of the composition.

When the water is included in an amount of less than 70 wt%, cellulose and agarose do not dissolve well in water, making it difficult to prepare the hydrogel. In addition, when the water is included in an amount exceeding 90 wt%, the hydrogel does not cure sufficiently, or mechanical properties such as modulus and toughness are significantly degraded, resulting in physical properties different from actual organs, which makes it difficult to use as an artificial organ or artificial tissue.

Accordingly, it is preferable that the water be included in an amount of 70 to 90 wt% based on the total weight of the composition, and more specifically, based on the total weight of the composition, it may be 70 wt% or more, 71 wt% or more, 72 wt% or more, 73 wt% or more, 74 wt% or more, 75 wt% or more, 76 wt% or more, 77 wt% or more, 78 wt% or more, 79 wt% or more, 80 wt% or more, or 90 wt% or less, 89 wt% or less, 88 wt% or less, 87 wt% or less, 86 wt% or less, 85 wt% or less, 84 wt% or less, 83 wt% or less, 82 wt% or less, 81 wt% or less, or 80 wt% or less.

According to one embodiment of the present invention, a hydrogel composition for an ultrasonography simulation can be used to construct tissues of an artificial kidney, artificial liver, artificial small intestine, artificial large intestine, artificial stomach, artificial gallbladder, artificial blood vessel, artificial bladder, artificial heart, artificial lung, artificial uterus, artificial skin, lymph nodes, and lesions.

In particular, a hydrogel composition for an ultrasonography simulation can be advantageously used to construct tissues of artificial kidneys, artificial skin, lymph nodes, and lesions.

According to one embodiment of the present invention, the artificial organ may include tissue formed from the hydrogel composition for an ultrasonography simulation. The artificial organ is implemented so that each tissue is distinguishable upon ultrasonography observation, allowing it to be observed like an actual human organ or tissue.

The artificial organ may be an artificial kidney, artificial liver, artificial small intestine, artificial large intestine, artificial stomach, artificial gallbladder, artificial blood vessel, artificial bladder, artificial heart, artificial lung, artificial uterus, artificial skin, or lymph node, but is not limited to these examples.

According to one embodiment of the present invention, a preparation method for a hydrogel composition for an ultrasonography simulation may comprise the steps of:
(a) dissolving 10 to 25 wt% of acrylamide and 1 to 4 wt% of agarose in 70 to 90 wt% of water based on the total weight of the composition;
(b) adding 0.001 to 5.0 wt% of cellulose to the dissolved solution of the step (a) and dispersing it; and
(c) adding 0.005 to 0.03 wt% of N,N-methylenebisacrylamide (MBA) and 0.05 to 0.10 wt% of ammonium persulfate (APS) to the dispersed solution of the step (b) and dissolving them.

The preparation method for a hydrogel composition for an ultrasonography simulation has a problem in that when the thermosetting agent, ammonium persulfate, is mixed all at once into a high-temperature agarose solution, the reaction begins before other materials are mixed or occurs before all materials in the solution are uniformly mixed. Therefore, to resolve this problem, the preparation method of the present invention is characterized by performing step (c) after steps (a) and (b) to prepare the hydrogel composition for an ultrasonography simulation.

Since the preparation method can be applied identically to the description regarding the hydrogel composition for an ultrasonography simulation mentioned above, redundant content is omitted.

To aid in understanding the present invention, preferred examples are provided below. However, the following examples are merely illustrative of the present invention, and it is obvious to those skilled in the art that various changes and modifications are possible within the scope and spirit of the present invention, and it is natural that such changes and modifications fall within the scope of the appended claims.

### Example 1: Preparation of hydrogel composition for ultrasonography simulation

### (1)Preparation of monomer solution

4.8 g of acrylamide (AM) and 0.8 g of agarose were added to 20 ml of distilled water (DI) and dissolved thoroughly to prepare a monomer solution.

### (2) Preparation of cellulose dispersion

0.01 g of cellulose was added to the monomer solution and uniformly dispersed to prepare a cellulose dispersion.

### (3) Preparation of hydrogel composition

A hydrogel composition was prepared by adding N,N-methylenebisacrylamide (MBA) and ammonium persulfate (APS) to the cellulose dispersion and dissolving them.

### Examples 2 to 10: Preparation of hydrogel composition for an ultrasonography simulation

A hydrogel composition for an ultrasonography simulation was prepared in the same manner as the Example 1, except that the content of each component is included as shown in Table 1 below.

**[Table 1]**

| | DI (ml) | AM (g) | Agarose (g) | MBA(g) | APS (g) | Cellulose (g) |
|---|---|---|---|---|---|---|
| Example 1 | 20 | 4.8 | 0.8 | 0.0046 | 0.0215 | 0.01 |
| Example 2 | 20 | 4.8 | 0.8 | 0.0046 | 0.0215 | 0.05 |
| Example 3 | 20 | 4.8 | 0.8 | 0.0046 | 0.0215 | 0.25 |
| Example 4 | 20 | 4.8 | 0.8 | 0.0046 | 0.0215 | 1.0 |
| Example 5 | 20 | 4.8 | 0.5 | 0.0038 | 0.0248 | 0.001 |
| Example 6 | 20 | 4.8 | 0.5 | 0.0038 | 0.0248 | 0.01 |
| Example 7 | 20 | 4.8 | 0.5 | 0.0038 | 0.0248 | 0.05 |
| Example 8 | 20 | 4.8 | 0.5 | 0.0038 | 0.0248 | 0.1 |
| Example 9 | 20 | 4.8 | 0.5 | 0.0038 | 0.0248 | 0.5 |
| Example 10 | 20 | 4.8 | 0.5 | 0.0038 | 0.0248 | 1.0 |

### Comparative Example

A hydrogel composition for an ultrasonography simulation was prepared in the same manner as in the Example 1, except that it does not contain cellulose.

### Test Example: Ultrasonic image of hydrogel for ultrasonography simulation

The hydrogel compositions for an ultrasonography simulation prepared in the Examples 1 to 4 and Comparative Example were poured into a mold and cured at 70°C for 20 minutes to produce a hydrogel for ultrasonography simulation. An ultrasonographic image of the hydrogel is shown in FIG. 2.

Looking at FIG. 2, it was confirmed that the hydrogels of Examples 1 to 4 containing cellulose appeared whiter in the ultrasonic image due to increased reflected sound compared to the hydrogel of Comparative Example that does not contain cellulose. In addition, it was found that as the content of cellulose increased, the hydrogels appeared whiter in the ultrasonic image.

In addition, the hydrogel compositions for an ultrasonography simulation prepared in the Examples 1, 5, and 8 were each poured into molds and cured at 70°C for 20 minutes to produce hydrogels for ultrasonography simulation. An ultrasonographic image of the hydrogel is shown in FIG. 3.

Looking at FIG. 3, as a result of observing ultrasonic images by stacking hydrogels prepared in Examples 1, 5, and 8 with different cellulose contents, it was found that as the content of cellulose increased, the ultrasonic image appeared whiter.

## Claims

1. A hydrogel composition for an ultrasonography simulation comprising a hydrogel monomer and a water-dispersible fibrous material,
wherein the content of the fibrous material is 0.001 wt% to 5.0 wt% based on the total weight of the composition.

2. The hydrogel composition for an ultrasonography simulation of claim 1, wherein the hydrogel monomer is at least one selected from acrylamide, agarose, methacrylamide, bis-acrylamide, N-N-dimethylacrylamide, N,N-methylenebisacrylamide, polyaniline, acrylic acid, methacrylic acid, vinylsulfonic acid, styrenesulfonic acid, polyacrylic acid, allylamine, methyl methacrylate, poly(ethylene glycol) methacrylate, poly(ethylene glycol) diacrylate, sodium polyacrylate, N-vinylpyrrolidone, imide, amide, agarose, gelatin, gelatin methacrylate, collagen, fibrinogen, fibrin, methyl cellulose, chitosan, chitin, polyethylene glycol, polyvinyl alcohol, carrageenan, 2-acrylamido-2-methylpropane-sulfonic acid, alginate, 2-hydroxyethyl methacrylate, and mixtures thereof.

3. The hydrogel composition for an ultrasonography simulation of claim 1, wherein the fibrous material is at least one selected from cellulose, cellulose nanofibers, glass fibers, and silicon oxide (SiO₂).

4. The hydrogel composition for an ultrasonography simulation of claim 1, wherein the composition comprises 10 to 25 wt% of acrylamide, 1 to 4 wt% of agarose, 0.001 to 5.0 wt% of cellulose, 0.005 to 0.03 wt% of N,N-methylenebisacrylamide (MBA), 0.05 to 0.10 wt% of ammonium persulfate (APS), and 70 to 90 wt% of water, based on the total weight of the composition.

5. An artificial organ comprising a tissue formed from the hydrogel composition for an ultrasonography simulation according to any one of claims 1 to 4.

6. The artificial organ of claim 5, wherein the artificial organ is an artificial kidney, artificial liver, artificial small intestine, artificial large intestine, artificial stomach, artificial gallbladder, artificial blood vessel, artificial bladder, artificial heart, artificial lung, artificial uterus, artificial skin, or lymph node.

7. A preparation method for a hydrogel composition for an ultrasonography simulation comprising the steps of:
(a) dissolving 10 to 25 wt% of acrylamide and 1 to 4 wt% of agarose in 70 to 90 wt% of water, based on the total weight of the composition;
(b) adding 0.001 to 5.0 wt% of cellulose to the dissolved solution of the step (a) and dispersing it; and
(c) adding 0.005 to 0.03 wt% of N,N-methylenebisacrylamide (MBA) and 0.05 to 0.10 wt% of ammonium persulfate (APS) to the dispersed solution of the step (b) and dissolving them.
